# EUROPÄISCHE PATENTSCHRIFT

(11) **EP 0 676 381 B1**
(45) Veröffentlichungstag und Bekanntmachung des Hinweises auf die Patenterteilung: **27.11.1996**
(21) Anmeldenummer: 95104884.2
(22) Anmeldetag: 01.04.1995
(51) Int. Cl.: C04B 35/64, B28B 3/00

(54) **Verfahren zur Herstellung eines Implantates**
Process for the production of an implant
Procédé pour la fabrication d'un implant

(30) Priorität: 11.04.1994 DE 4412324
(43) Veröffentlichungstag der Anmeldung: 11.10.1995
(73) Patentinhaber: Kalawrytinos, Georg, Dr.-Ing., D-52223 Stolberg (DE)
(72) Erfinder: Kalawrytinos, Georg, Dr.-Ing., D-52223 Stolberg (DE); Sax, Hans Michael, cand. Ing., D-68535 Edingen/Neckarhausen (DE); Zografou, Constantin, Dr.-Ing., D-52072 Aachen (DE); Telle, Rainer, Prof. Dr., D-52068 Aachen (DE)
(74) Vertreter: Bauer, Hubert, Dipl.-Ing.

(56) Entgegenhaltungen:
- EP-A- 0 176 266
- EP-A- 0 403 743
- DE-A- 2 738 808

## Beschreibung

Die Erfindung betrifft ein Verfahren zur Herstellung eines implantierbaren Körpers aus Phosphat- oder Oxid- oder dergleichen Keramikpulver, das in eine Form gebracht, darin verdichtet, anschließend als Grünling entformt und schließlich gesintert wird.

Obschon konventionelle Gießverfahren nach wie vor am häufigsten zur Herstellung komplizierter Körper angewendet werden, sind auch bereits verschiedene Verfah ren allgemein bekannt, die eine endkonturennahe Fertigung von Formteilen nach dem sogenannten Near Net Shape Processing ermöglichen. Diese Verfahren bieten mehrere Vorteile:

Fertigungstechnisch ist bei diesen Verfahren das Aufschmelzen des Formkörpermaterials entbehrlich, da bei niedrigeren Temperaturen als die bei Schmelzverfahren üblichen gearbeitet werden kann. Auch das schließlich durchzuführende Sintern eines Grünlings, um die erforderliche Festigkeit und ein möglichst porenfreies Zusammenbacken der Pulverpartikel zu erreichen, erfordert nur ausnahmsweise Temperaturen im Schmelzbereich.

Vorrichtungstechnisch sind bei derartigen Verfahren keine aufwendigen Maschinen und gekühlte Stahlformen erforderlich, wie sie ansonsten zur Herstellung von Kleinteilen hoher Oberflächengüte benötigt werden. Würde man sich statt dessen mit dem konventionellen Formsandbau begnügen, ließen sich weder Formteile mit glatter Oberfläche noch mit feinen Strukturen herstellen, so daß unter Umständen umfangreiche Nachbearbeitungen erfolgen müßten.

Nach einem allgemein bekannten Verfahren zur endkonturennahen Fertigung der eingangs beschriebenen Art wird das Pulver unter hohem Druck einachsig mittels eines Kolbens in eine Form gepreßt oder auch mit Hilfe einer geteilten Form mehrachsig durch hohen Druck allseitig zusammengepreßt. Um die Poren des so erzeugten Grünlings zu verdichten, wird dieser anschließend bei Temperaturen gesintert, die in der Regel deutlich unterhalb der Schmelztemperatur des Pulvers liegen.

Das vorbeschriebene Verfahren hat sich jedoch insbesondere wegen der aufwendigen Verpressung des Pulvers nicht durchsetzen können. Auch verteuert sich dabei die Herstellung der Formkörper durch Verluste an Pulver, was einen sehr hohen Kostenanteil verursacht.

Aus der Offenlegungsschrift DE-A-2738808 ist ein Verfahren zur Herstellung von Formkörpern aus Keramik bekannt, bei dem mit Hilfe eines elastischen Modells Formkörper gleich in ihrer end gültigen Kontur gepreßt werden konnen.

Zur Herstellung von Formkörpern, die besonders feine und homogene Mikrostrukturen aufweisen sollen, oder bei denen eine schwierige Nachbearbeitung vermieden werden soll, ist auch schon ein sogenanntes Metallpulver-Gießverfahren bekannt. Dazu wird eine Spritzgießmaschine eingesetzt, mit der eine plastische Mischung aus Pulver und Bindemittel bei etwa 150 ° in eine Form gespritzt wird. Nachteilig bei diesem Verfahren sind die hohen Formkosten wie auch die erforderlichen großen Bindemittelmengen, die zudem besonders lange Bindemittelentfernungszeiten erfordern, bevor der getrocknete Grünling in einem Ofen unter Teilvakuum aufgeheizt werden kann, um den restlichen Binder zu zersetzen und erst sodann die Sinterung des Grünlings zu ermöglichen.

Der Erfindung liegt die Aufgabe zugrunde, ein Verfahren der eingangs beschriebe nen Art so auszugestalten, daß es zu seiner Durchführung einen nur geringen Vorrichtungsaufwand erfordert und insbesondere auf teure Formen verzichtet werden kann und auch die Sinterung des Grünlings unmittelbar nach dessen Entformung zuläßt.

Zur Lösung dieser Aufgabe wird von einem Verfahren der im Oberbegriff des Anspruchs 1 genannten Art ausgegangen und erfindungsgemäß vorgeschlagen, entsprechend den im kennzeichnenden Teil des Anspruchs 1 angegebenen Merkmalen zu verfahren.

Nach dem erfindungsgemäßen Verfahren wird also eine bestimmte Menge Keramikpulver, die zu dem bekannten Volumen des herzustellenden Körpers in einem bestimmten Verhältnis, das in jedem Falle größer als 1 : 1 ist und einfach ermittelt werden kann, unter dreidimensionaler Aufweitung des Formhohlraumes in diesen eingegeben und darin vorverdichtet. Durch den kaltisostatischen Preßvorgang wird der Formhohlraum entsprechend den erforderlichen Abmessungen des dabei entstehenden Grünlings verkleinert. Dieser weist sodann noch eine derartige Überdimensionierung auf, die der durch den nachfolgenden Sintervorgang verursachten Schwindung entspricht, so daß nach der Sinterung ein mit den geforderten Endkonturen übereinstimmender Körper vorliegt, der keiner Nachbearbeitung bedarf.

Nach einer besonders vorteilhaften Ausgestaltung des erfindungsgemäßen Verfahrens wird eine aus Kautschuk hergestellte Form verwendet, die nach ihrer Befüllung mit und Aufweitung durch Keramikpulver verschlossen und in eine Folie eingeschweißt und sodann in das Ölbad eines Druckbehälters gegeben wird. Darin erfolgt die kaltisostatische Verdichtung mit einem Preßdruck von 100 - 300 MPa, vorzugsweise von 200 MPa.

Durch Versuche konnte nachgewiesen werden, daß sich höhere Preßdrücke nicht empfehlen, da diese eine Entformung des Grünlings erschweren. So wurde festgestellt, daß eine Ablösung des Preßlings von der Form nach einem Preßdruck von 800 MPa nahezu unmöglich ist. Falls es jedoch erforderlich sein sollte, doch mit höheren Preßdrücken zu arbeiten, um die Grünlingsdichte zu steigern, so empfiehlt sich ein Vorpressen mit einem Preßdruck von 200 MPa innerhalb der aus Silikon hergestellten Form und einer anschließenden kaltisostatischen Nachverpressung des entformten Grünlings unter höherem Preßdruck.

Vorzugsweise erfolgt die kaltisostatische Verpressung, indem die mit vorverdichtetem Pulver ausgefüllte Form in eine doppelte Polyäthylenfolie eingeschweißt ist. Dabei dient die innere Folie dazu, die Luft aus der Form zu entfernen und weiterhin dazu, ebenso wie die äußere Folie, das Eindringen von Öl zu verhindern. Da sich Verunreinigungen nachteilig auf die spätere Qualität des herzustellenden Körpers im Einsatz als Implantat auswirken, bietet die äußere Folienhülle zusätzlich die Möglichkeit, sauberer zu arbeiten.

Nach einer weiteren Ausgestaltung des erfindungsgemäßen Verfahrens wird der kaltisostatische Preßdruck 2 bis 5 min, vorzugsweise 3 min, aufrechterhalten und anschließend um 2 MPa/sec bis zur vollständigen Entlastung abgesenkt.

Durch die relativ langsame Druckabsenkung werden Spannungen im Grünkörper vermieden, die ansonsten zu seiner Zerstörung führen könnten.

Nach einer weiteren Ausgestaltung des erfindungsgemäßen Verfahrens wird der Grünling drucklos unter atmospährischen Bedingungen aufrecht auf einer Al₂O₃-Platte und durch ebensolche Platten seitlich und nach oben abgeschirmt in einem Muffelofen gesintert.

Diese Muffeltechnik gewährleistet einen schonenden Sinterbrand und verhindert eine direkte Bestrahlung des Grünlings. Eine direkte Bestrahlung würde sich negativ auf den Grünling auswirken, da dadurch Spannungen hervorgerufen werden würden, die Spannungsrisse verursachen können. Die vorgeschlagene Abmufflung ist deshalb dringend zu empfehlen.

Vorzugsweise wird für den Sintervorgang ein Temperaturzeitverlauf gewählt, bei dem die Aufheizgeschwindigkeit bis zur jeweiligen Endtemperatur (welche zwischen 900 °C und 1.500 °C liegt) sowie die Haltezeit (welche zwischen 30 und 120 min liegt) so gewählt, daß die gewünschten physikalischen Keimwerte des Werkstoffes eingestellt werden.

## Patentansprüche

1. Verfahren zur Herstellung eines implantierbaren Körpers aus Phosphat- oder Oxid- oder dergleichen Keramikpulver, das in eine Form eingebracht, darin verdichtet, anschließend als Grünling entformt und schließlich gesintert wird, dadurch gekennzeichnet, daß in die dreidimensional veränderbare Form eine bestimmte Menge Keramikpulver eingegeben und darin unter Aufweitung des Formhohlraumes vorverdichtet wird, worauf der Formhohlraum durch einen kaltisostatischen Preßvorgang so verengt wird, daß ein solcher Grünling entsteht, dessen Abmessungen derartig überdimensioniert sind, daß nach der durch den Sintervorgang verursachten Schwindung ein Körper mit vorherbestimmten Endabmessungen vorliegt.

2. Verfahren nach Anspruch 1, dadurch gekennzeichnet, daß eine aus Kautschuk hergestellte Form verwendet wird, die nach ihrer Befüllung mit und Aufweitung durch Keramikpulver verschlossen und in eine Folie eingeschweißt und sodann in das Ölbad eines Druckbehälters gegeben wird, worin die kaltisostatische Verdichtung mit einem Preßdruck von 100 bis 300 MPa, vorzugsweise von 200 MPa, erfolgt.

3. Verfahren nach Anspruch 1 oder 2, dadurch gekennzeichnet, daß der kaltisostatische Preßdruck 2 bis 5 min, vorzugsweise drei min, aufrechterhalten wird und anschließend um 2 MPa/sec bis zur vollständigen Entlastung abgesenkt wird.

4. Verfahren nach einem der Ansprüche 1 bis 3, dadurch gekennzeichnet, daß der Grünling drucklos unter atmosphärischen Bedingungen aufrecht auf einer Al₂O₃-Platte stehend und durch ebensolche Platten seitlich und nach oben abgeschirmt in einem gemuffelten Elektroofen gesintert wird.

5. Verfahren nach einem der Ansprüche 1 bis 4, dadurch gekennzeichnet, daß ein Temperaturverlauf für den Sintervorgang gewählt wird, bei dem die Aufheizgeschwindigkeit 2 K/min bis zum Erreichen der jeweiligen Endtemperatur im Sinterintervall beträgt, sowie eine zwischen 30 - 120 min liegende Haltezeit und eine Abheizgeschwindigkeit von 5 K/min gewählt wird.

## Claims

1. Process for manufacturing an implantable body of a phosphate or oxide or the like ceramic powder, which is entered into a mould where it is compressed, subsequently removed as a rawling and finally sintered, characterised in that into the three-dimensionally changeable form is fed a specified amount of ceramic powder and pre-compressed therein whilst expanding the hollow space of the mould, whereafter the hollow space is narrowed by a cold isostatic compression process so that a rawling is produced the dimensions of which over overdimensioned in such a manner that, after shrinkage caused by the sintering process, a body of predetermined final dimensions is obtained.

2. Process according to Claim 1, **characterised in that** a mould of caoutchouc is used which, after being filled with and expanded by ceramic powder, is sealed and welded into a foil and then delivered into the oil bath of a pressure vessel, wherein cold isostatic compression is carried out at a compression pressure between 100 and 300 MPa, preferably 200 MPa.

3. Process according to Claim 1 or 2, **characterised in that** the cold isostatic compression pressure is maintained for between 2 and 5 mins, preferably 3 min, and thereafter reduced to 2 MPa/sec until fully reduced.

4. Process according to one of Claims 1 to 3, **characterised in that** the rawling is sintered in a muffled electric furnace free of pressure under atmospheric conditions whilst standing on an Al₂O₃ plate and being laterally and upwardly screened by such plates.

5. Process according to one of Claims 1 to 4, **characterised in that** a temperature course for the sintering process is chosen in which the heating-up speed is 2 K/min until reaching a respective end temperature in a sintering interval, and a residence time between 30 and 120 min and a heating-off speed of 5 K/min.

## Revendications

1. Procédé pour la fabrication d'un corps implantable à partir d'une poudre de phosphate ou d'oxyde, ou d'une poudre céramique de ce genre, qui est introduite dans un moule dans lequel elle est comprimée pour être ensuite démoulée sous forme d'une ébauche compactée et, finalement, vitrifiée, ce procédé étant caractérisé par le fait qu'une quantité déterminée de poudre céramique est versée dans un moule adapté pour se modifier dans les trois dimensions et subit dans ce moule une compression préalable qui provoque un agrandissement de la cavité de ce dernier, après quoi, par un procédé de compression isostatique à froid, cette cavité du moule est rétrécie, de manière à obtenir une ébauche dont les dimensions soient surdimensionnées, afin que suite au retrait provoqué par le processus de vitrification, on obtienne un corps ayant des dimensions finales déterminées au préalable.

2. Procédé selon la revendication 1, caractérisé par le fait que l'on utilise un moule fabriqué à partir de caoutchouc, lequel moule, après avoir été rempli avec la poudre de céramique et agrandi par celle-ci, est fermé et soudé dans une feuille, puis placé dans le bain d'huile d'un récipient sous pression, dans lequel la compression isostatique à froid a lieu sous une pression de compression allant de 100 à 300 MPa, mais de préférence de 200 MPa.

3. Procédé selon l'une ou l'autre des revendications 1 ou 2, caractérisé par le fait que la pression de compression isostatique à froid est maintenue pendant 2 à 5 min, de préférence trois minutes, et qu'elle est ensuite abaissée de 2 MPa/sec jusqu'à la réduction totale de la pression.

4. Procédé selon l'une quelconque des revendications 1 à 3, caractérisé par le fait que l'ébauche est placée debout, sur une plaque d'alumine ou oxyde d'aluminium (Al₂O₃) et protégée, au-dessus et sur tous ses côtés, par des plaques de même composition, est vitrifiée dans un four électrique à moufle, sans pression et sous les conditions atmosphériques.

5. Procédé selon l'une quelconque des revendications 1 à 4, caractérisé par le fait que l'on choisit pour le processus de vitrification une courbe de température par laquelle la vitesse de mise en température est égale à 2 K/min jusqu'à l'obtention de la température finale respective dans l'intervalle de vitrification, et que l'on choisit un temps de pause compris entre 30 et 120 min et une vitesse de refroidissement de 5 K/min.
